# FASCICULE DE BREVET EUROPEEN

(11) **EP 0 939 619 B1**
(45) Date de publication et mention de la délivrance du brevet: **15.01.2003**
(21) Numéro de dépôt: 97909389.5
(22) Date de dépôt: 10.10.1997
(51) Int. Cl.: A61K 7/50

(54) **COMPOSITIONS COSMETIQUES DETERGENTES ET UTILISATION**
KOSMETISCHES REINIGUNGSMITTEL UND SEINE ANWENDUNG
DETERGENT COSMETIC COMPOSITIONS AND USE THEREOF

(30) Priorité: 15.11.1996 FR 9613977; 18.03.1997 FR 9703280
(43) Date de publication de la demande: 08.09.1999
(73) Titulaire: L'OREAL, 75008 Paris (FR)
(72) Inventeur: CAUWET-MARTIN, Danièle, F-75011 Paris (FR)
(74) Mandataire: Le Blainvaux Bellegarde, Françoise
(86) Numéro de dépôt international: FR9701815
(87) Numéro de publication internationale: WO98022087

(56) Documents cités:
- EP-A- 0 007 120
- EP-A- 0 355 368
- EP-A- 0 692 236
- MANUFACTURING CHEMIST, vol. 60, no. 1, janvier 1989, WOOLWICH, pages 38-40, XP000026532

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, une base lavante constituée de tensioactifs à pouvoir détergent dans laquelle est également présent à titre d'agents conditionneurs une nanoémulsion définie ci-dessous. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Dans ce but, on a déjà proposé d'utiliser les huiles végétales ou animales. Compte tenu du caractère insoluble des huiles utilisables dans les compositions de lavage et de conditionnement, on cherche à maintenir les huiles en dispersion régulière dans le milieu sans cependant faire chuter la viscosité et les propriétés détergentes et moussantes des compositions. Les huiles doivent également être véhiculées sur les matières kératiniques traitées en vue de leur conférer, suivant l'application, des propriétés de douceur, de brillance et de démêlage sans conférer de caractère gras.

Ainsi, à la suite d'importantes recherches menées sur la question, il a maintenant été trouvé par la Demanderesse, qu'en utilisant (A) une base lavante et (B) un système conditionneur comprenant au moins une émulsion huile-dans-eau ayant des globules d'huile dont la taille moyenne est inférieure à 150 nm et comprenant une phase lipidique amphiphile qui comprend au moins un lipide amphiphile non-ionique liquide à une température ambiante inférieure à 45°C, il est possible d'obtenir des compositions détergentes stables présentant d'excellentes propriétés cosmétiques, en particulier la facilité de coiffage, le maintien, la nervosité et le volume des cheveux traités et ceci tout en conservant leur bon pouvoir lavant intrinsèque et en particulier leur pouvoir moussant.

Ces nouvelles compositions permettent de déposer une quantité plus importante d'huile sur les cheveux qu'avec une émulsion classique, mais sans toucher ou aspect visuel gras.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse sans aucune sensation de gras.

Ainsi, la présente invention a pour objet de nouvelles compositions détergentes et conditionnantes caractérisée par le fait qu'elles comprennent, dans un milieu aqueux cosmétiquement acceptable, (A) une base lavante et (B) un système conditionneur comprenant au moins une émulsion huile-dans-eau ayant des globules d'huile dont la taille moyenne est inférieure à 150 nm et comprenant une phase lipidique amphiphile, cette phase lipidique amphiphile comprenant au moins un lipide amphiphile non-ionique liquide à une température ambiante inférieure à 45°C.

L'invention a également pour objet l'utilisation en cosmétique des compositions ci-dessus pour le nettoyage et /ou le démaquillage et/ou le conditionnement des matières kératiniques telles que les cheveux et la peau.

Comme indiqué précédemment, les éléments essentiels rentrant dans la composition des produits selon l'invention sont (A) une base lavante, (B) un système conditionneur comprenant au moins une émulsion huile-dans-eau ayant des globules d'huile dont la taille moyenne est inférieure à 150 nm et comprenant une phase lipidique amphiphile, caractérisée par le fait que la phase lipidique amphiphile comprend au moins un lipide amphiphile non-ionique liquide à une température ambiante inférieure à 45°C.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotéres, non ioniques et cationiques.

Toutefois, selon l'invention, la base lavante comprend de préférence des tensioactifs anioniques ou des mélanges de tensioactifs anioniques et de tensioactifs amphotères, et encore plus préférentiellement ne contient que ce type de tensioactif ou mélange de tensioactifs.

La quantité minimale de base lavante est celle juste suffisante pour conférer à la composition finale un pouvoir moussant et/ou détergent satisfaisant, alors que des quantités trop importantes de base lavante n'apportent pas vraiment d'avantages supplémentaires.

Ainsi, selon l'invention, la base lavante peut représenter de 4 % à 50 % en poids, de préférence de 10 % à 35 % en poids, et encore plus préférentiellement de 12 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensio-actifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les aminés grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'aminés tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement béta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensio-actifs et en particulier des mélanges d'agents tensio-actifs anioniques et d'agents tensio-actifs amphotères ou non ioniques. Un mélange particulièrement préféré est un mélange constitué d'au moins un agent tensio-actif anionique et d'au moins un agent tensio-actif amphotère.

On utilise de préférence un agent tensio-actif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène. le cocoyl iséthionate de sodium et l'alphaoléfine(C₁₄-C₁₆) sulfonate de sodium et leurs mélange avec :
- soit un agent tensio-actif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensio-actif amphotére de type zwittérionique tel que les alkylbétaïnes en particulier la cocoylbétaïne commercialisée sous la dénomination "DEHYTON AB 30" en solution aqueuse à 32 % de MA par la société HENKEL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

### B- SYSTEME CONDITIONNEUR

Les compositions selon l'invention comprennent nécessairement comme système conditionneur une émulsion huile-dans-eau ayant des globules d'huile dont la taille moyenne est inférieure à 150 nm et comprenant une phase lipidique amphiphile, caractérisée par le fait que la phase lipidique amphiphile comprend au moins un lipide amphiphile non-ionique liquide à une température ambiante inférieure à 45°C.

Les lipides amphiphiles non-ioniques de l'invention sont préférentiellement choisis parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée. On peut également utiliser les mélanges des composés ci-dessus.

Les tensioactifs siliconés utilisables selon la présente invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.

De préférence, le tensioactif siliconé utilisé selon la présente invention est un composé de formule (I) : 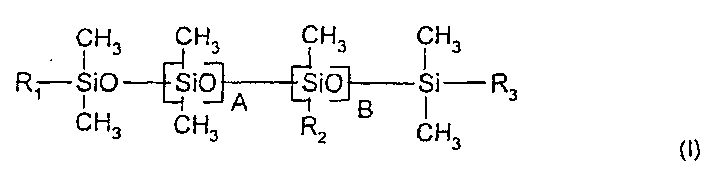 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 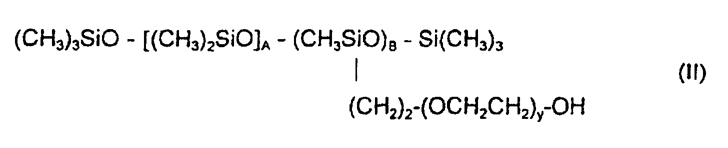 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO - (OCH₂CH₂)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A est 15 et y est 13.

Parmi les lipides amphiphiles non ioniques, on peut plus particulièrement citer, à titre d'exemple :
- l'isostéarate de polyéthylèneglycol de poids moléculaire 400,
- l'isostéarate de diglycéryle,
- le laurate de polyglycérol comportant 10 unités de glycérol,
- l'oléate de sorbitane,
- l'isostéarate de sorbitane,
- le cocoate d'α-butylglucoside ou le caprate d'α-butylglucoside.

Le rapport pondéral de la quantité d'huile contenue dans l'émulsion conforme à l'invention sur la quantité de la phase lipidique amphiphile varie, de préférence, de 2 à 10 et plus préférentiellement de 3 à 6.

Une forme particulière d'émulsion conforme à l'invention est caractérisée par le fait que la phase lipidique amphiphile comprend en plus un ou plusieurs lipides amphiphiles ioniques.

Les lipides amphiphiles ioniques, utilisés dans les nanoémulsions de l'invention, sont choisis, de préférence, dans le groupe formé par les lipides anioniques, les lipides amphotères et les lipides cationiques et leurs mélanges.

Les lipides amphiphiles anioniques sont plus particulièrement choisis dans le groupe formé par :
- les sels alcalins du dicétyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques tels que ceux de formule : 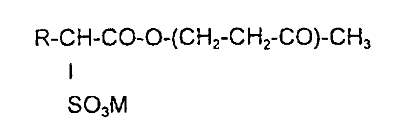
dans laquelle R représente des radicaux alkyle en C₁₆-C₂₂, en particulier les radicaux C₁₆H₃₃ et C₁₈H₃₇, pris en mélange ou séparément et M est un métal alcalin tel que le sodium.

Les lipides amphiphiles cationiques, utilisés dans les nanoémulsions de l'invention, sont choisis, de préférence, dans le groupe formé par les sels d'ammonium quaternaire, les amines grasses et leurs sels.

Les sels d'ammonium quaternaires sont par exemple :
- ceux qui présentent la formule générale (VIII) suivante : 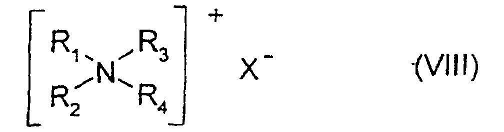
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (IX) suivante : 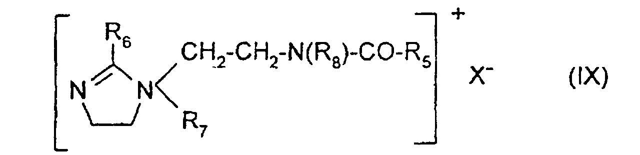
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates.

De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (X) : 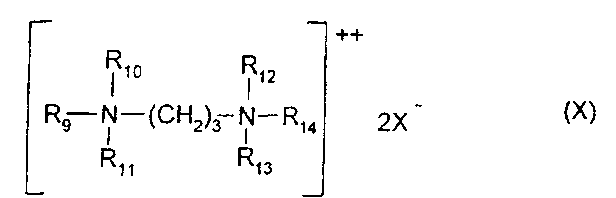
dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (XI) suivante : 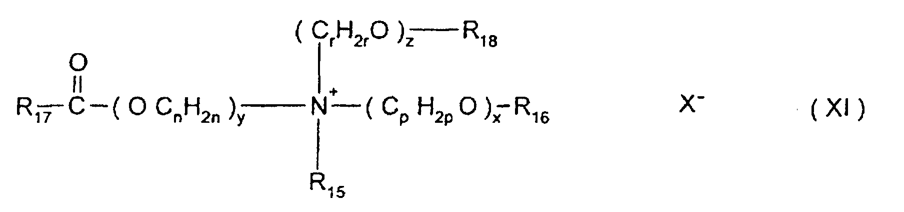 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :

- le radical 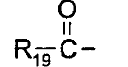
- les radicaux R₂₀ hydrocarbonés en C₁-C₂₂ linéaires ou ramifiés, saturés ou insaturés,
- l'atome d'hydrogène,
- R₁₈ est choisi parmi :

- le radical 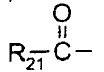
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (XI) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 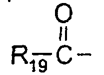
   - les radicaux méthyle, éthyle ou hydrocarbonés en C₁₄-C₂₂
   - l'atome d'hydrogène ;
- R₁₈ est choisi parmi :
   - le radical 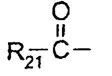
   - l'atome d'hydrogène ;
R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés. Avantageusement, les radicaux hydrocarbonés sont linéaires.

On peut citer par exemple les composés de formule ( XI ) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents,

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsutfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VIII) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Selon l'invention, le chlorure de béhényltriméthylammonium est le sel d'ammonium quaternaire le plus particulièrement préféré.

Les lipides ioniques amphiphiles sont présents dans les nanoémulsions de l'invention, de préférence, dans des concentrations allant de 0 à 60% en poids et plus particulièrement de 10 à 50 % en poids par rapport au poids total de la phase lipidique amphiphile.

Les nanoémulsions conformes à l'invention comportent une quantité d'huile allant de préférence, de 5 à 40% en poids par rapport au poids total de l'émulsion et de préférence de 10 à 30% en poids.

Les huiles pouvant être utilisées dans les émulsions de l'invention sont choisies préférentiellement dans le groupe formé par :
- les huiles animales ou végétales formées par des esters d'acides gras et de polyols, en particulier les triglycérides liquides, par exemple les huiles de tournesol, d'avocat, de maïs, de soja, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée ramifiée contenant de 3 à 30 atomes de carbone, par exemple, l'huile de Purcellin ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine ;
- des carbures halogénés, notamment des fluorocarbures tels que des fluoramines par exemple la perfluorotributylamine, des hydrocarbures fluorés, par exemple le perfluorodécahydronaphtalène, des fluoroesters et des fluoroethers ;
- des esters d'acide minéral et d'un alcool ;
- des éthers et des polyéthers ;
- des silicones en mélange avec au moins l'une des huiles définies ci-dessus, par exemple le décaméthylcyclopentasiloxane ou le dodécaméthylcyclohexasiloxane.

La taille moyenne des globules d'huile est généralement comprise entre 30 et 150 nm, de préférence entre 40 et 100 nm et encore plus particulièrement entre 50 et 80 nm.

Les compositions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines telles que la vitamine E et ses dérivés, les provitamines telles que le panthénol, les humectants, les céramides, les pseudocéramides et les filtres solaires.

Les compositions selon l'invention contiennent généralement une émulsion telle que définie ci-dessus dans des concentrations comprises entre 2 et 50% en poids par rapport au poids total de la composition.

La ou les huiles peuvent être utilisées dans les compositions conformes à l'invention dans des concentrations généralement comprises entre 0,1 et 15 %, et de préférence entre 0,2 et 10 % en poids par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol. le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensio-actifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones volatiles ou non volatiles, solubles et insolubles dans le milieu et leurs mélanges.

Les agents conditionneurs de type polymères cationiques utilisables conformément à la présente invention peuvent être choisis parmi tous ceux déjà connus en soi comme améliorant les - propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.

Selon l'invention, le ou les polymères cationiques peuvent représenter de 0.001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association (base lavante + nanoémulsion d'huile) conforme à l'invention ne soient pas, ou substantiellement pas, altérées par la ou les adjonctions envisagées.

Ces compositions peuvent se présenter sous la forme de liquides plus ou moins épaissis, de crèmes ou de gels et elles conviennent principalement au lavage, au soin des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatif, illustrant l'invention vont maintenant être donnés.

### EXEMPLE

On a réalisé deux compositions de shampooing, l'une conforme à l'invention (composition A) et l'autre comparative (composition B) :

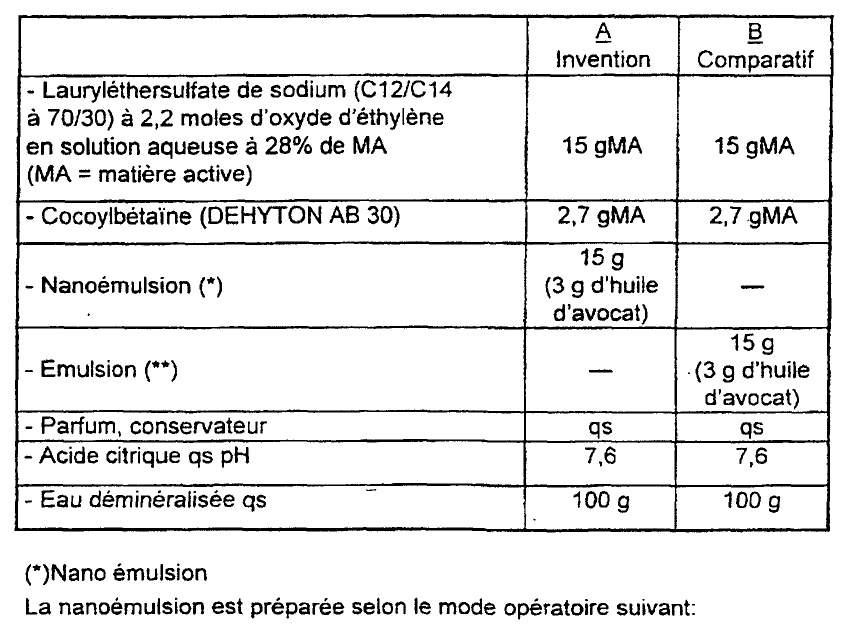

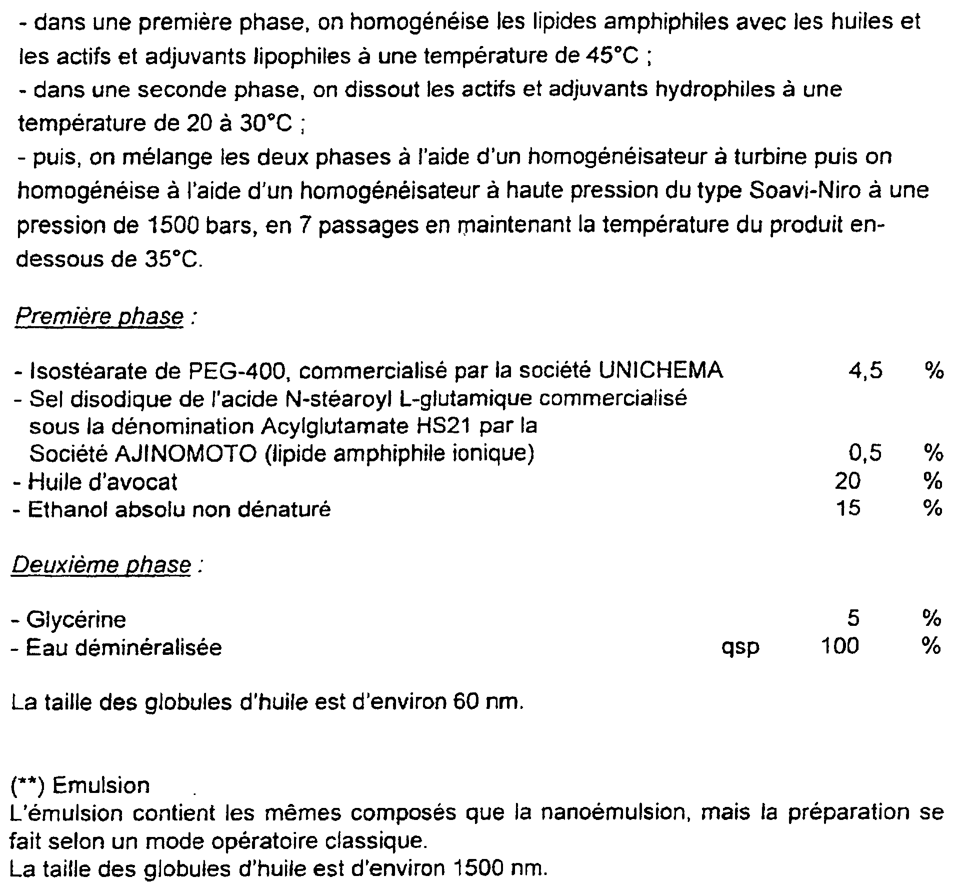

Les compositions de shampooing sont préparées en mélangeant l'émulsion ou la nanoémulsion avec les autres constituants du shampooing à température ambiante.

La composition B est instable et l'huile relargue à la surface du liquide alors que la composition A est homogène et stable.

La composition B présente un pouvoir moussant insuffisant alors que la composition A selon l'invention présente un excellent pouvoir moussant.

On a effectué un shampooing en appliquant environ 12 g de la composition A sur des cheveux sensibilisés préalablement mouillés. On fait mousser le shampooing puis on rince abondamment à l'eau.

On procède selon le même mode opératoire que ci-dessus avec la composition comparative B.

Un panel d'experts a évalué l'aspect des cheveux séchés.

Tous les experts indiquent que les cheveux traités avec la composition A selon l'invention sont bien individualisés, non collés alors que les cheveux traités avec la composition B ont un aspect sales et gras.

## Revendications

1. Compositions détergentes et conditionnantes, **caractérisées par le fait qu'**elles comprennent, dans un milieu aqueux cosmétiquement acceptable, (A) une base lavante et (B) un système conditionneur comprenant au moins une émulsion huile-dans-eau ayant des globules d'huile dont la taille moyenne est inférieure à 150 nm et comprenant une phase lipidique amphiphile, cette phase lipidique amphiphile comprenant au moins un lipide amphiphile non-ionique liquide à une température ambiante inférieure à 45°C.

2. Compositions selon la revendication 1, **caractérisées par le fait que** ladite base lavante comprend un ou plusieurs tensioactifs choisis parmi des tensioactifs anioniques, amphotères, non ioniques, cationiques et leurs mélanges.

3. Compositions selon la revendication 1 ou 2, **caractérisées par le fait que** ladite base lavante est présente à une teneur pondérale comprise entre 4 % et 50 % en poids par rapport au poids total de la composition, de préférence comprise entre 10 % et 35 % en poids et plus préférentiellement comprise entre 12 % et 25 % en poids.

4. Compositions selon l'une des revendications 1 à 3, **caractérisées par le fait que** le lipide amphiphile non-ionique est choisi parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée, et leurs mélanges.

5. Compositions selon la revendication 4, **caractérisées en ce que** le tensioactif siliconé est un composé de formule (I) : 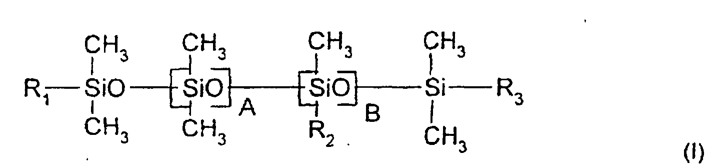 dans laquelle
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

6. Compositions selon la revendication 4, **caractérisées en ce que** le tensioactif siliconé est un composé de formule (II) : 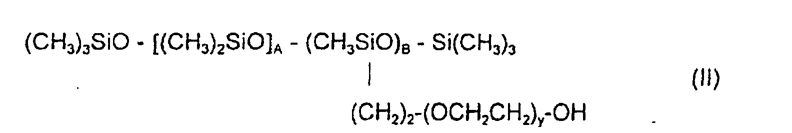 dans laquelle A est un nombre entier allant de 20 à 105, B est un nombre entier allant de 2 à 10 et y est un nombre entier allant de 10 à 20.

7. Compositions selon la revendication 4, **caractérisées en ce que** le tensioactif siliconé est un composé de formule (III) :
HO - (OCH₂CH₂)_{y}-(CH₂)₃ - [(CH₃)₂SiO]_{A'} - (CH₂)₃ - (OCH₂CH₂)_{y} - OH (III)
dans laquelle A' et y sont des nombres entiers allant de 10 à 20.

8. Compositions selon l'une quelconque des revendications précédentes, **caractérisées en ce qu'**elles comprennent, en outre, au moins un lipide amphiphile ionique.

9. Compositions selon la revendication précédente, **caractérisées en ce que** le lipide amphiphile ionique est choisi dans le groupe formé par les lipides anioniques, les lipides amphotères, les lipides cationiques et leurs mélanges.

10. Compositions selon la revendication 9, **caractérisées par le fait que** le lipide amphiphile cationique est choisi dans le groupe formé par les sels d'ammonium quaternaire et les amines grasses.

11. Compositions selon la revendication 10, **caractérisées par le fait que** les sels d'ammonium quaternaire sont choisis dans le groupe formé par :
- les sels d'ammonium quaternaire de formule générale (VIII) suivante : 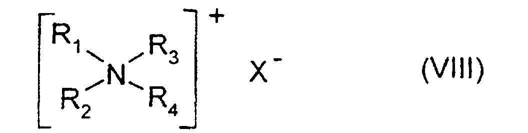
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium,
- les sels de diammonium quaternaire de formule (X) : 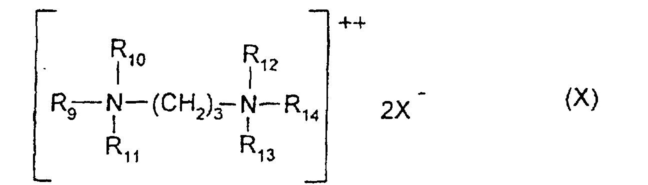 dans laquelle R₉ désigne un radical aliphatique comportant environ de 16 à 30 atomes de carbone, R₁₀, R₁₁, R₁₂, R₁₃, et R₁₄ sont choisis parmi l'hydrogène ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

12. Compositions selon l'une quelconque des revendications 8 à 11, **caractérisées par le fait que** le lipide amphiphile ionique est présent dans des concentrations allant de 0 à 60 % en poids par rapport au poids total de la phase lipidique amphiphile, et de préférence de 10 à 50% en poids.

13. Compositions selon l'une des revendications 1 à 12, **caractérisées par le fait que** le rapport pondéral de la quantité d'huile sur la quantité en phase lipidique amphiphile est compris entre 2 et 10 inclus.

14. Compositions selon l'une quelconque des revendications 1 à 13, **caractérisées par le fait qu'**elles comprennent une proportion d'huile allant de 5 à 40 % en poids par rapport au poids total de l'émulsion, de préférence de 10 à 30 % en poids.

15. Compositions selon l'une quelconque des revendications 1 à 14, **caractérisées par le fait que** l'huile est présente dans des concentrations comprises entre 0,1 et 15 %, et de préférence entre 0,2 et 10 % en poids par rapport au poids total de la composition.

16. Composition selon l'une quelconque des revendications 1 à 15, **caractérisée par le fait qu'**elle contient en plus un ou plusieurs adjuvants destinés à améliorer les propriétés cosmétiques choisis par les tensio-actifs cationiques, les polymères anioniques ou non ioniques ou cationiques ou amphotères, les protéines, les céramides, les pseudocéramides ou les silicones.

17. Composition selon l'une quelconque des revendications 1 à 16, **caractérisée par le fait que** les globules d'huile ont une taille moyenne allant de 30 à 150 nm.

18. Utilisation d'une composition telle définie dans l'une quelconque des revendications 1 à 17 pour le nettoyage et/ou le démaquillage et/ou le conditionnement des matières kératiniques.

19. Procédé de lavage et de conditionnement des matières kératiniques telles que les cheveux consistant à appliquer sur lesdites matières mouillées une quantité efficace d'une composition telle que définie dans l'une quelconque des revendications 1 à 17, puis à effectuer un rinçage à l'eau après un éventuel temps de pause.

## Patentansprüche

1. Reinigende und konditionierende Zusammensetzungen, **dadurch gekennzeichnet, daß** sie in einem kosmetisch akzeptablen, wäßrigen Medium (A) eine reinigende Basisformulierung und (B) ein Konditioniersystem enthalten, das mindestens eine Öl-in-Wasser-Emulsion enthält, deren Ölkügelchen eine mittlere Größe unter 150 nm aufweisen und die eine amphiphile Lipidphase enthält, wobei die amphiphile Lipidphase mindestens ein nichtionisches amphiphiles Lipid enthält, das bei einer Umgebungstemperatur unter 45 °C flüssig ist.

2. Zusammensetzungen nach Anspruch 1, **dadurch gekennzeichnet, daß** die reinigende Basisformulierung einen oder mehrere grenzflächenaktive Stoffe enthält, die unter den anionischen, amphoteren, nichtionischen und kationischen grenzflächenaktiven Stoffen und deren Gemischen ausgewählt sind.

3. Zusammensetzungen nach Anspruch 1 oder 2, **dadurch gekennzeichnet, daß** die reinigende Basisformulierung in einem Gewichtsanteil von 4 bis 50 Gew.-%, bezogen auf das Gesamtgewicht der Zusammensetzung, vorzugsweise 10 bis 35 Gew.-% und noch bevorzugter 12 bis 25 Gew.-% vorliegt.

4. Zusammensetzungen nach einem der Ansprüche 1 bis 3, **dadurch gekennzeichnet, daß** das nichtionische amphiphile Lipid unter den siliconhaltigen grenzflächenaktiven Stoffen und den Estern mindestens eines Polyols, das unter Polyethylenglykol mit 1 bis 60 Ethylenoxideinheiten, Sorbitan, Glycerin mit 2 bis 30 Ethylenoxideinheiten und Polyglycerinen mit 2 bis 15 Glycerineinheiten ausgewählt ist, und mindestens einer Fettsäure, die mindestens eine gesättigte oder ungesättigte, geradkettige oder verzweigte C₈₋₂₂-Alkylkette enthält, und deren Gemischen ausgewählt ist.

5. Zusammensetzungen nach Anspruch 4, **dadurch gekennzeichnet, daß** der siliconhaltige grenzflächenaktive Stoff eine Verbindung der Formel (I) ist: 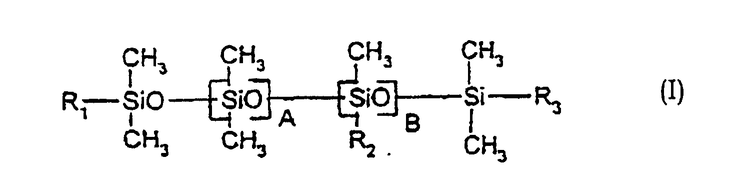 worin bedeuten:
die Gruppen R₁, R₂ und R₃ unabhängig voneinander C₁₋₆-Alkyl oder eine Gruppe -(CH₂)ₓ - (OCH₂CH₂)_{y} - (OCH₂CH₂CH₂)_{z} - OR₄, wobei mindestens eine der Gruppen R₁, R₂ oder R₃ keine Alkylgruppe bedeutet; und wobei R₄ Wasserstoff, Alkyl oder Acyl ist;
A 0 oder eine ganze Zahl von 1 bis 200;
B 0 oder eine ganze Zahl von 1 bis 50, mit der Maßgabe, daß A und B nicht gleichzeitig Null sind;
x eine ganze Zahl von 1 bis 6;
y eine ganze Zahl von 1 bis 30;
z 0 oder eine ganze Zahl von 1 bis 5.

6. Zusammensetzungen nach Anspruch 5, **dadurch gekennzeichnet, daß** der siliconhaltige grenzflächenaktive Stoff eine Verbindung der Formel (II) ist: 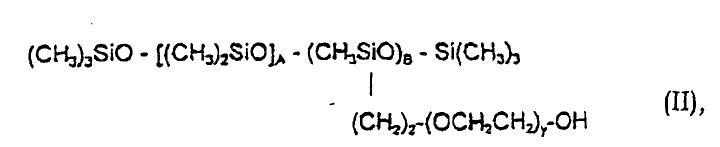 worin A eine ganze Zahl von 20 bis 105, B eine ganze Zahl von 2 bis 10 und y eine ganze Zahl von 10 bis 20 bedeutet.

7. Zusammensetzungen nach Anspruch 4, **dadurch gekennzeichnet, daß** der siliconhaltige grenzflächenaktive Stoff eine Verbindung der Formel (III) ist:
HO - (OCH₂CH₂)_{y}-(CH₂)₃ - [(CH₃)₂SiO]_{A'} - (CH₂)₃ - (OCH₂CH₂)_{y} - OH (III),
worin A' und y ganze Zahlen von 10 bis 20 bedeuten.

8. Zusammensetzungen nach einem der vorhergehenden Ansprüche, **dadurch gekennzeichnet, daß** sie ferner mindestens ein ionisches amphiphiles Lipid enthalten.

9. Zusammensetzungen nach dem vorhergehenden Anspruch, **dadurch gekennzeichnet, daß** das ionische amphiphile Lipid unter den anionischen Lipiden, amphoteren Lipiden, kationischen Lipiden und deren Gemischen ausgewählt ist.

10. Zusammensetzungen nach Anspruch 9, **dadurch gekennzeichnet, daß** das kationische amphiphile Lipid unter den quartären Ammoniumsalzen und den Fettaminen ausgewählt ist.

11. Zusammensetzungen nach Anspruch 10, **dadurch gekennzeichnet, daß** die quartären Ammoniumsalze ausgewählt sind unter:
- den quartären Ammoniumsalzen der folgenden allgemeinen Formel (VIII): 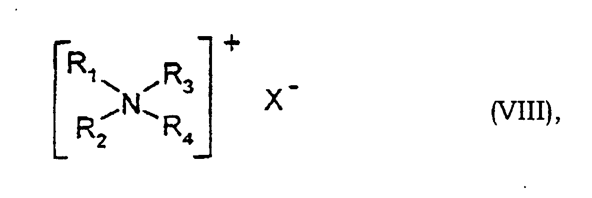
worin die Gruppen R₁ bis R₄, die identisch oder voneinander verschieden sein können, ein geradkettige oder verzweigte aliphatische Gruppe mit 1 bis 30 Kohlenstoffatomen oder eine aromatische Gruppe, wie Aryl oder Alkylaryl, bedeuten und X ein Anion ist, das unter den Halogeniden, Phosphaten, Acetaten, Lactaten, C₂₋₆-Alkylsulfaten und Alkyl- oder Alkylarylsulfonaten ausgewählt ist;
- den quartären Ammoniumsalzen von Imidazolinium;
- den quartären Diammoniumsalzen der Formel (X): 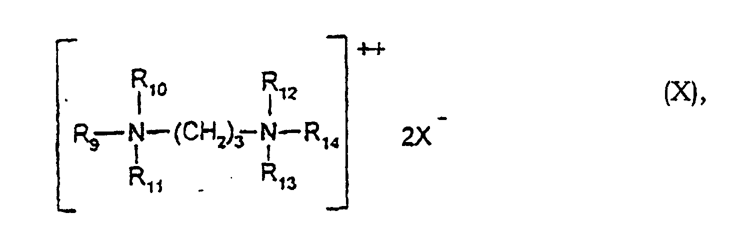
worin R₉ eine aliphatische Gruppe mit etwa 16 bis 30 Kohlenstoffatomen bedeutet, die Gruppen R₁₀, R₁₁, R₁₂, R₁₃ und R₁₄ unter Wasserstoff oder den Alkylgruppen mit 1 bis 4 Kohlenstoffatomen ausgewählt sind und X ein Anion ist, das unter den Halogeniden, Acetaten, Phosphaten, Nitraten und Methylsulfaten ausgewählt ist; und
- den quartären Ammoniumsalzen, die mindestens eine Estergruppe enthalten.

12. Zusammensetzungen nach einem der Ansprüche 8 bis 11, **dadurch gekennzeichnet, daß** das ionische amphiphile Lipid in Konzentrationen von 0 bis 60 Gew.-%, bezogen auf das Gesamtgewicht der amphiphilen Lipidphase, und vorzugsweise 10 bis 50 Gew.-% vorliegt.

13. Zusammensetzungen nach einem der Ansprüche 1 bis 12, **dadurch gekennzeichnet, daß** das Gewichtsverhältnis der Menge des Öls und der Menge der amphiphilen Lipidphase im Bereich von 2 bis 10 liegt.

14. Zusammensetzungen nach einem der Ansprüche 1 bis 13, **dadurch gekennzeichnet, daß** sie einen Ölanteil von 5 bis 40 Gew.-%, bezogen auf das Gesamtgewicht der Emulsion, und vorzugsweise von 10 bis 30 Gew.-% enthalten.

15. Zusammensetzungen nach einem der Ansprüche 1 bis 14, **dadurch gekennzeichnet, daß** das Öl in Konzentrationen von 0,1 bis 15 Gew.-% und vorzugsweise 0,2 bis 10 Gew.-%, bezogen auf das Gesamtgewicht der Zusammensetzung, vorliegt.

16. Zusammensetzungen nach einem der Ansprüche 1 bis 15, **dadurch gekennzeichnet, daß** sie ferner einen oder mehrere Zusatzstoffe enthalten, die dazu vorgesehen sind, die kosmetischen Eigenschaften zu verbessern, und die unter den kationischen grenzflächenaktiven Stoffen, anionischen, nichtionischen, kationischen oder amphoteren Polymeren, Proteinen, Ceramiden, Pseudoceramiden oder Siliconen ausgewählt sind.

17. Zusammensetzungen nach einem der Ansprüche 1 bis 16, **dadurch gekennzeichnet, daß** die Ölkügelchen eine mittlere Größe von 30 bis 150 nm aufweisen.

18. Verwendung einer Zusammensetzung nach einem der Ansprüche 1 bis 17 zur Reinigung und/oder zum Abschminken und/oder zur Konditionierung von Keratinsubstanzen.

19. Verfahren zum Waschen und Konditionieren von Keratinsubstanzen, wie dem Haar, das darin besteht, auf die feuchten Keratinsubstanzen eine wirksame Menge einer Zusammensetzung nach einem der Ansprüche 1 bis 17 aufzutragen und anschließend, gegebenenfalls nach einer Einwirkzeit, mit Wasser zu spülen.

## Claims

1. Detergent and conditioning compositions, **characterized in that** they comprise, in a cosmetically acceptable aqueous medium, (A) a washing base and (B) a conditioning system comprising at least one oil-in-water emulsion having oil globules with a mean size of less than 150 nm and comprising an amphiphilic lipid phase, this amphiphilic lipid phase comprising at least one non-ionic amphiphilic lipid which is liquid at an ambient temperature of less than 45°C.

2. Compositions according to Claim 1, **characterized in that** the said washing base comprises one or more surfactants chosen from anionic, amphoteric, non-ionic and cationic surfactants and mixtures thereof.

3. Compositions according to Claim 1 or 2, **characterized in that** the said washing base is present at a content by weight of between 4% and 50% by weight with respect to the total weight of the composition, preferably of between 10% and 35% by weight and more preferentially of between 12% and 25% by weight.

4. Compositions according to one of Claims 1 to 3, **characterized in that** the non-ionic amphiphilic lipid is chosen from silicone surfactants and esters of at least one polyol chosen from the group formed by polyethylene glycol containing from 1 to 60 ethylene oxide units, sorbitan, glycerol containing from 2 to 30 ethylene oxide units or polyglycerols containing from 2 to 15 glycerol units and of at least one fatty acid containing at least one linear or branched, saturated or unsaturated, C₈-C₂₂ alkyl chain, and mixtures thereof.

5. Compositions according to Claim 4, **characterized in that** the silicone surfactant is a compound of formula (I): 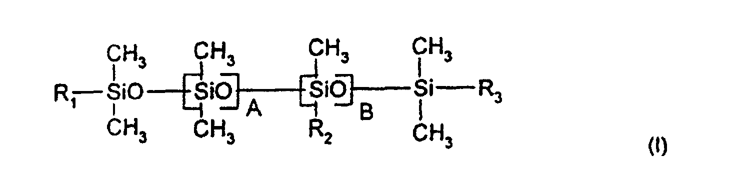 in which
R₁, R₂ and R₃, independently of one another, represent a C₁-C₆ alkyl radical or a -(CH₂)ₓ-(OCH₂CH₂)_{y}-(OCH₂CH₂CH₂)_{z}-OR₄ radical, at least one R₁, R₂ or R₃ radical not being an alkyl radical, R₄ being a hydrogen, an alkyl radical or an acyl radical;
A is an integer ranging from 0 to 200;
B is an integer ranging from 0 to 50; provided that A and B are not simultaneously equal to zero;
x is an integer ranging from 1 to 6;
y is an integer ranging from 1 to 30;
z is an integer ranging from 0 to 5.

6. Compositions according to Claim 4, **characterized in that** the silicone surfactant is a compound of formula (II): 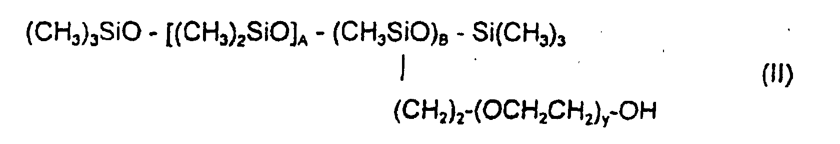 in which A is an integer ranging from 20 to 105, B is an integer ranging from 2 to 10 and y is an integer ranging from 10 to 20.

7. Compositions according to Claim 4, **characterized in that** the silicone surfactant is a compound of formula (III):
HO-(OCH₂CH₂)_{y}-(CH₂)₃-[(CH₃)₂SiO]_{A'}-(CH₂)₃-(OCH₂CH₂)_{y}-OH (III)
in which A' and y are integers ranging from 10 to 20.

8. Compositions according to any one of the preceding claims, **characterized in that** they additionally comprise at least one ionic amphiphilic lipid.

9. Compositions according to the preceding claim, **characterized in that** the ionic amphiphilic lipid is chosen from the group formed by anionic lipids, amphoteric lipids, cationic lipids and mixtures thereof.

10. Compositions according to Claim 9, **characterized in that** the cationic amphiphilic lipid is chosen from the group formed by quaternary ammonium salts and fatty amines.

11. Compositions according to Claim 10, **characterized in that** the quaternary ammonium salts are chosen from the group formed by:
- the quaternary ammonium salts of following general formula (VIII): 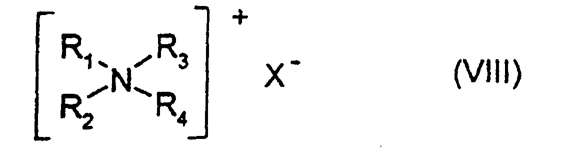 in which the R₁ to R₄ radicals, which can be identical or different, represent a linear or branched aliphatic radical containing from 1 to 30 carbon atoms, or an aromatic radical, such as aryl or alkylaryl. X is an anion chosen from the group of the halides, phosphates, acetates, lactates, (C₂-C₆)alkyl sulphates, or alkyl- or alkylarylsulphonates,
- imidazolinium quaternary ammonium salts,
- the quaternary diammonium salts of formula (X): 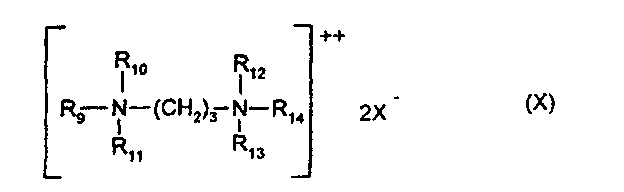 in which R₉ denotes an aliphatic radical containing approximately from 16 to 30 carbon atoms, R₁₀, R₁₁, R₁₂, R₁₃ and R₁₄ are chosen from hydrogen or an alkyl radical containing from 1 to 4 carbon atoms and X is an anion chosen from the group of the halides, acetates, phosphates, nitrates and methyl sulphates,
- quaternary ammonium salts containing at least one ester functional group.

12. Compositions according to any one of Claims 8 to 11, **characterized in that** the ionic amphiphilic lipid is present in concentrations ranging from 0 to 60% by weight with respect to the total weight of the amphiphilic lipid phase and preferably from 10 to 50% by weight.

13. Compositions according to one of Claims 1 to 12, **characterized in that** the ratio by weight of the amount of oil to the amount of amphiphilic lipid phase is between 2 and 10 inclusive.

14. Compositions according to any one of Claims 1 to 13, **characterized in that** they comprise a proportion of oil ranging from 5 to 40% by weight with respect to the total weight of the emulsion, preferably from 10 to 30% by weight.

15. Compositions according to any one of Claims 1 to 14, **characterized in that** the oil is present in concentrations of between 0.1 and 15% and preferably between 0.2 and 10% by weight with respect to the total weight of the composition.

16. Composition according to any one of Claims 1 to 15, **characterized in that** it additionally contains one or more adjuvants intended to improve the cosmetic properties chosen from cationic surfactants, anionic or non-ionic or cationic or amphoteric polymers, proteins, ceramides, pseudoceramides or silicones.

17. Composition according to any one of Claims 1 to 16, **characterized in that** the oil globules have a mean size ranging from 30 to 150 nm.

18. Use of a composition as defined in any one of Claims 1 to 17 for cleaning and/or removing make-up from and/or conditioning keratinous substances.

19. Process for washing and conditioning keratinous substances, such as hair, which consists in applying, to the said wetted substances, an effective amount of a composition as defined in any one of Claims 1 to 17 and in then rinsing with water, after an optional period of rest.
